# EUROPEAN PATENT APPLICATION

(11) **EP 4 582 044 A1**
(43) Date of publication of application: **09.07.2025**
(21) Application number: 23860865.7
(22) Date of filing: 30.08.2023
(51) Int. Cl.: A61B 34/30, A61B 34/00, A61B 17/16, A61B 17/00

(54) **CALIBRATION MOUNTING PART-EQUIPPED SURGICAL DEVICE FOR SURGICAL ROBOT**

(30) Priority: 31.08.2022 KR 20220110118
(71) Applicant: Curexo, Inc., Seoul 05814 (KR)
(72) Inventor: LEE, Chang Hun, Seoul 05814 (KR); LEE, Kwan Ju, Seoul 05814 (KR)
(74) Representative: Stöckeler, Ferdinand
(86) International application number: PCT/KR2023/012860
(87) International publication number: WO 2024/049189

(57) **Abstract**

Disclosed is a calibration mounting part-equipped surgical device for a surgical robot, and more particularly to a calibration mounting part-equipped surgical device for a surgical robot, in which various medical tools are interchangeably installed and used, and a calibration tool is easily installed to perform accurate calibration. The surgical device for a surgical robot includes: a sleeve supporting a medical tool comprising a shaft; a chuck device part coupling with the sleeve comprising the shaft; a holder part installed with the chuck device part and mounted to a distal end of a robot arm; and a calibration mounting part detachably provided in the sleeve and installed with a calibration tool.

## Description

### [TECHNICAL FIELD]

The disclosure relates to a calibration mounting part-equipped surgical device for a surgical robot, and more particularly to a calibration mounting part-equipped surgical device for a surgical robot, in which various medical tools are interchangeably installed and used, and a calibration tool is easily installed to perform accurate calibration.

### [BACKGROUND ART]

In general, surgical treatment of joint diseases includes arthroscopic surgery, cartilage cell transplantation, etc., and artificial joint surgery is performed in the case of severe diseases. Artificial joint surgery representatively includes manual artificial joint surgery performed by medical personnel, and artificial joint surgery using a robot.

Artificial joint surgery using the robot refers to a surgical procedure that cuts off the kneecap and installs an artificial knee joint (implant) by rotating a cutter of a cutting device mounted to a distal end of a position-variable arm of the robot based on information input to a computer, and employs a cutting device including a cutting tool such as a cutter (hereinafter referred to as a 'cutter').

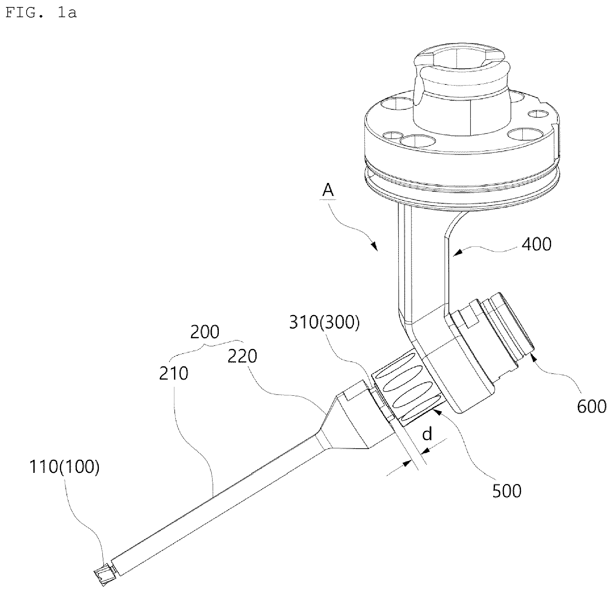

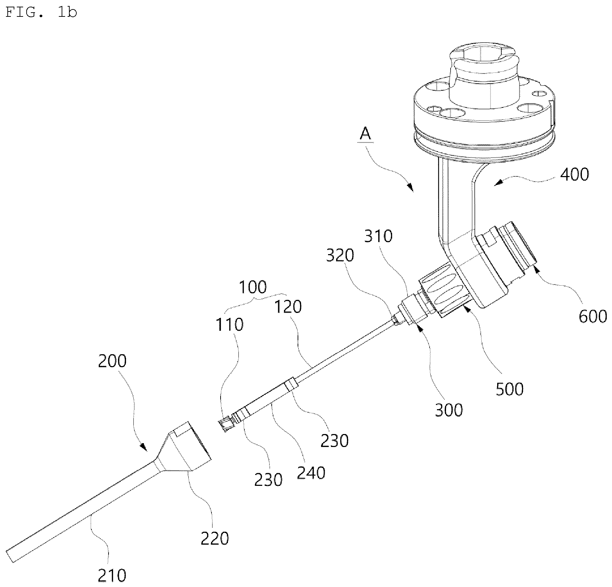

FIG. 1A is a perspective view showing an assembled state to illustrate a conventional cutting device for a surgical robot, and FIG. 1B is an exploded perspective view to illustrate the conventional cutting device for the surgical robot.

Referring to FIGS. 1A and 1B, the conventional cutting device A for the surgical robot includes a sleeve 200 supporting a cutter 100 that performs a cutting operation, a chuck device part 300 holding the cutter, a holder part 400 installed with the chuck device part and mounted to the distal end of the robot's position-variable arm (not shown), an operating nut 500 applying an operation force to make the cutter be clamped to or unclamped from the chuck device part 300, and a motor (not shown) mounted to a motor coupling member 600 connected to the rear of the holder portion 400 to provide a rotational force to the cutter.

In more detail, the cutter includes a head 110 formed with cutting edges, and a shaft 120 formed extending from the head 110 and shaped like a round bar. The cutter 100 performs the cutting operation as the rear end of the shaft 120 is connected to a motor and rotated, the outer circumferential surface of the shaft 120 is rotatably supported by the sleeve 200 so that the shaft 120 is prevented from vibration or bending when rotating, and the head 110 protrudes outward from the sleeve 200 so that the cutting operation for a bone can be performed by the rotation of the head 110.

The sleeve 200 includes a pipe portion 210 having a small diameter, and a fastening cap 220 formed on one end of the pipe portion so as to be fastened to the chuck device part 300. In this sleeve, a bearing 230 rotatably supporting the shaft of the cutter, a spacer 240, etc. are inserted.

The chuck device part 300 refers to a component to perform a clamping operation or an unclamping operation by pressing the shaft of the cutter as chucks 320 (also referred to as collets, a part to hold the shaft of the cutter, usually 3 to 4 jaws that moves radially) provided in the front of the chuck body 310 are brought together or separated.

The foregoing conventional cutting device for the surgical robot performs a cutting operation while supporting the cutter 100, but has limitations that result in the following problems.

First, in the conventional cutting device for the surgical robot, if the length or diameter of the cutter 100 is changed depending on the method or type of the artificial joint surgery, the sleeve 200 suitable for that cutter should be individually manufactured, thereby causing problems making preparation and use difficult and increasing medical costs due to increased manufacturing costs.

For example, among artificial knee joint surgeries, total knee arthroplasty (TKA), which cuts both sides of the femur and tibia, usually uses a cutter having a relatively long shaft and a head diameter of 6.2 mm; Uni-knee Arthroplasty (UKA), which cuts only one side of the femur and tibia, uses a 5.0 mm cutter having a relatively short shaft; and Guide Hole Resection (GHR), which performs cutting (or sawing) by fixing a cutting block to a bone during surgery, uses a 3.2 mm cutter to drill an insertion hole for a fixing pin to fix that cutting block, thereby making preparation for surgery difficult. Accordingly, there is an urgent need for a sleeve having compatibility.

In particular, the conventional cutting device for the surgical robot has limitations that it is difficult to install and accurately position a calibration tool such as a marker to calibrate the end of the cutter during the artificial joint surgery using the robot every time when the length and diameter of the sleeve 200 are changed.

### [DOCUMENT OF RELATED ART]

### [PATENT DOCUMENT]

(Patent Document 1) Korean Patent No. 10-1624512, titled "CUTTING APPARATUS FOR CUTTING SYSTEM WHICH USES THE ROBOT"
(Patent Document 2) Korean Patent No. 10-0873014, titled "THE KNEE JOINT CUTTING SYSTEM WHICH USES THE ROBOT"

### [DISCLOSURE]

### [TECHNICAL PROBLEM]

The disclosure has been conceived to solve the problems described above, and an aspect of the disclosure is to provide a calibration mounting part-equipped surgical device for a surgical robot, in which various medical tools are interchangeably installed and used, and a calibration tool is easily installed to perform calibration accurately.

### [TECHNICAL SOLUTION]

According to an aspect of the disclosure, a calibration mounting part-equipped surgical device for a surgical robot according to the disclosure is characterized in including: a shaft; a chuck device part coupling with the sleeve including the shaft; a holder part installed with the chuck device part and mounted to a distal end of a robot arm; and a calibration mounting part detachably provided in the sleeve and installed with a calibration tool.

The calibration mounting part may include: a holding cap member fastened to the sleeve; and a calibration mounting member mounted to the sleeve by the holding cap member.

The calibration mounting member may include a bar-shaped calibration shaft in which a marker is installed, and a position-setting member formed in the calibration shaft and seated on the holding cap member.

The sleeve may include a hollow sleeve body formed with a coupling male-thread portion formed on an outer circumferential surface on one side, and a shaft insertion hole formed penetrating in a longitudinal direction.

The holding cap member may include a cap body shaped like a cap and connected to the hollow sleeve body, a rod insertion hole formed in the cap body to insert the calibration shaft therein, and a cap female thread formed on an inner circumferential surface of the cap body to be fastened to the coupling male-thread portion.

The calibration mounting part-equipped surgical device for a surgical robot according to the disclosure may further include a mounting member rotation blocking portion configured to block movement of the calibration mounting member.

The mounting member rotation blocking portion may include: a pin insertion groove formed in the sleeve; and a rotation blocking pin formed in the position-setting member and inserted in the pin insertion groove.

The pin insertion groove is structured to include a foreign substance discharging portion on one side to facilitate the discharge of foreign substances.

Preferably, the calibration mounting member may include a cap separation preventing means configured to prevent the holding cap member from being separated from the calibration shaft, the position-setting member may be shaped like a disc to be inserted in the holding cap member, the rotation blocking pin may include a plurality of rotation blocking pins protruding from the disc, and the holding cap member may be formed with an inspection opening to inspect an alignment state between the sleeve and the calibration mounting member in the cap body.

The sleeve may include a sleeve cap coupled to the shaft insertion hole corresponding to an outer free end of the hollow sleeve body; a plurality of first support bearings provided in the shaft insertion hole being in contact with the sleeve cap and supporting the shaft; a spacer formed having a cylindrical shape and provided being in contact with the first support bearing; a plurality of second support bearings provided being in contact with the spacer and supporting the shaft; and an airtight member inserted through a coupling female-thread portion formed inside the shaft insertion hole.

The shaft insertion hole includes a fitting groove to which the sleeve cap, the first and second support bearings, and the spacer are fitted; a locking protrusion to which the second support bearing is locked; and an airtight member insertion groove recessed at an inner end of the coupling female-thread portion and inserting the airtight member therein.

### [ADVANTAGEOUS EFFECTS]

As described above, a calibration mounting part-equipped surgical device for a surgical robot according to the disclosure has an effect on easily replacing and using various cutters because a sleeve includes a hollow sleeve body having a relatively large outer diameter rather than a conventional thin and long pipe and is thus interchangeably used without being replaced every time when the type of cutter is changed.

A calibration mounting part-equipped surgical device for a surgical robot according to the disclosure has an effect on performing simple and accurate calibration by assembling a calibration mounting part to a sleeve through a fastening method. In addition, when the rotation blocking pin formed in the position-setting member is inserted in the pin insertion groove of the sleeve during the process of assembling the calibration mounting part, the movement of the calibration mounting member installed with the calibration tool is fundamentally blocked, thereby having an advantage of enabling accurate calibration.

### [DESCRIPTION OF DRAWINGS]

FIGS. 1A and 1B are views for illustrating a conventional surgical device for an orthopedic surgical robot, in which FIG. 1A is a perspective view and FIG. 1B is an exploded perspective view.
FIG. 2 is a perspective view showing that a calibration tool is installed in a calibration mounting part-equipped surgical device for a surgical robot according to an embodiment of the disclosure.
FIG. 3 is an exploded perspective view for illustrating a calibration mounting part-equipped surgical device for a surgical robot according to an embodiment of the disclosure.
FIGS. 4A to 4C are views for illustrating a sleeve in a calibration mounting part-equipped surgical device for a surgical robot according to an embodiment of the disclosure, in which FIG. 4A is a perspective view, FIG. 4B is an overall cross-sectional view, and FIG. 4C is a cross-sectional view of a hollow sleeve body.
FIGS. 5A to 5C are views for illustrating a calibration mounting part in a calibration mounting part-equipped surgical device for a surgical robot according to an embodiment of the disclosure, in which FIG. 5A is a perspective view, FIG. 5B is an exploded perspective view, and FIG. 5C is a cross-sectional view.
FIGS. 6A to 6C are front views showing cutters used as surgical tools in a calibration mounting part-equipped surgical device for a surgical robot according to an embodiment of the disclosure,
FIGS. 7A to 7C are perspective views of a calibration mounting part-equipped surgical device for a surgical robot installed with cutters as surgical tools according to an embodiment of the disclosure.

### [MODE FOR INVENTION]

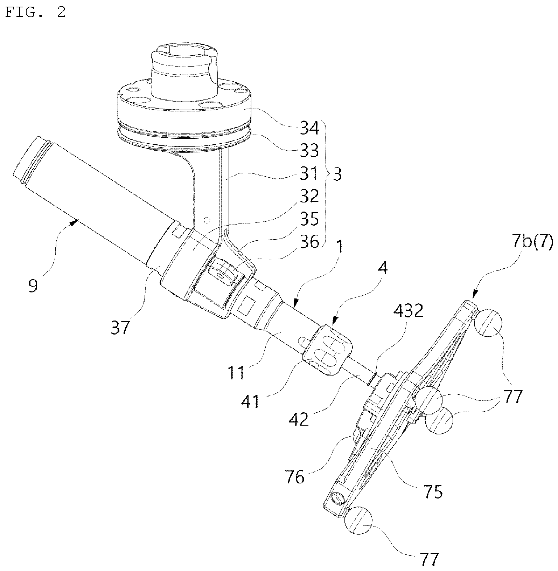

Below, exemplary embodiments of the disclosure will be described in detail with reference to the accompanying drawings, i.e., FIGS. 2 to 7C, in which like numerals refer to like components throughout FIGS. 2 to 7C.

Meanwhile, detailed descriptions of the configurations and their operations and effects, which can be easily understood by a person having ordinary knowledge in the art from general technologies in the accompanying drawings, will be simplified or omitted. Further, the disclosure is characterized in a calibration mounting part-equipped surgical device for a surgical robot, and thus related parts will be illustrated and described and the descriptions about the remaining parts will be simplified or omitted.

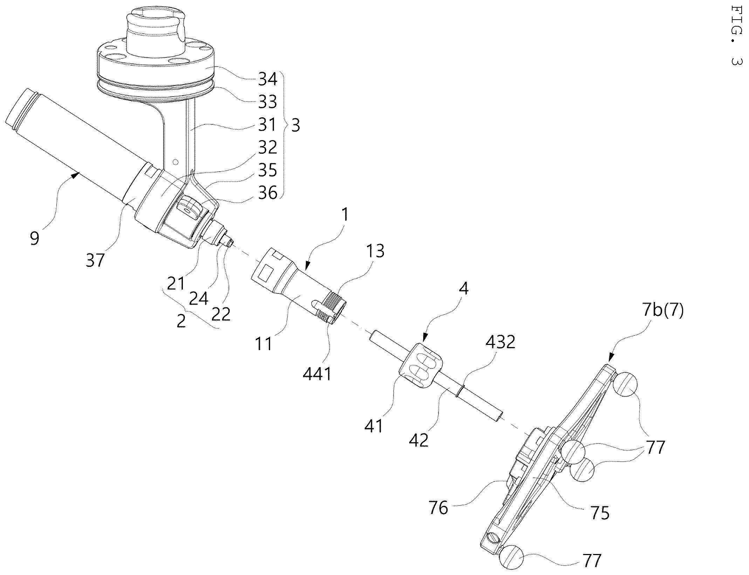

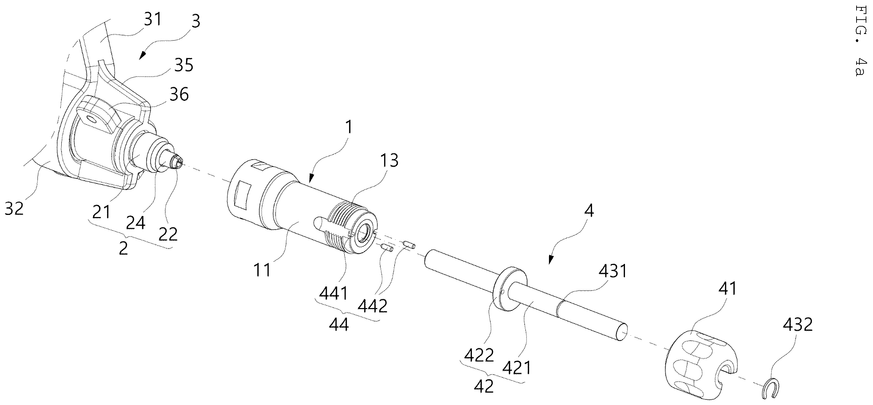

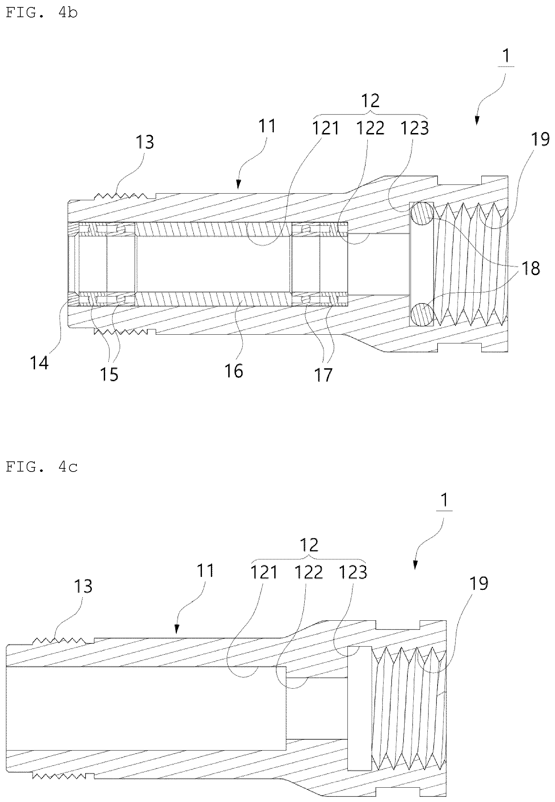

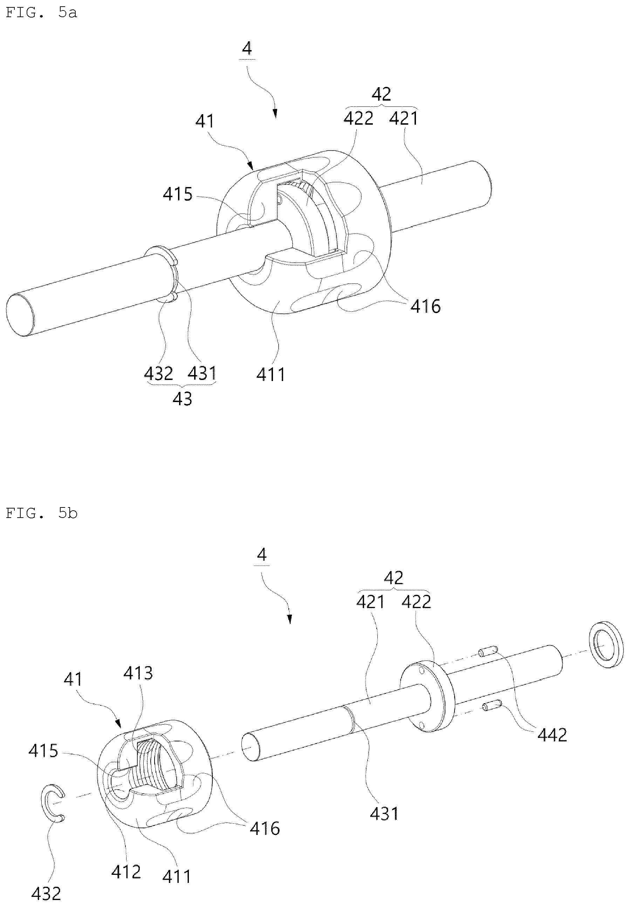

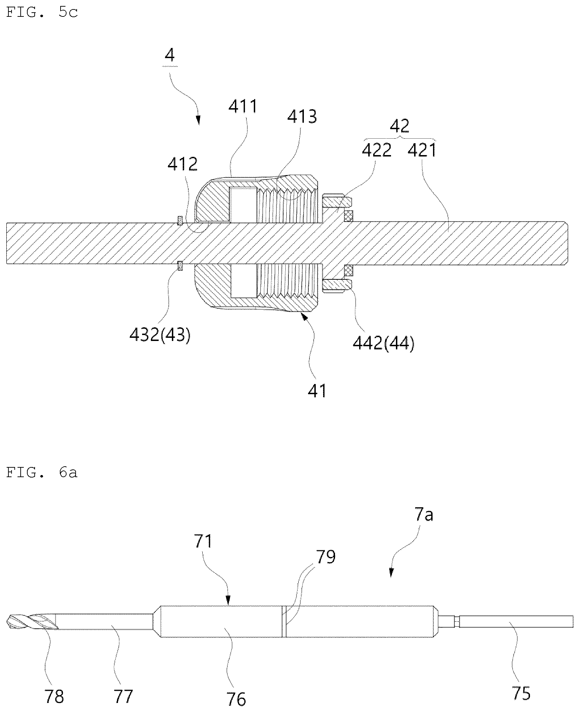

FIG. 2 is a perspective view showing that a calibration tool is installed in a calibration mounting part-equipped surgical device for a surgical robot according to an embodiment of the disclosure, FIG. 3 is an exploded perspective view for illustrating a calibration mounting part-equipped surgical device for a surgical robot according to an embodiment of the disclosure, FIGS. 4A to 4C are views for illustrating a sleeve in a calibration mounting part-equipped surgical device for a surgical robot according to an embodiment of the disclosure, in which FIG. 4A is a perspective view, FIG. 4B is an overall cross-sectional view, and FIG. 4C is a cross-sectional view of a hollow sleeve body. FIGS. 5A to 5C are views for illustrating a calibration mounting part in a calibration mounting part-equipped surgical device for a surgical robot according to an embodiment of the disclosure, in which FIG. 5A is a perspective view, FIG. 5B is an exploded perspective view, and FIG. 5C is a cross-sectional view.

Referring to FIGS. 2 to 5B, a surgical device for a surgical robot, provided with a calibration mounting part, according to an embodiment of the disclosure is configured to allow various medical tools to be interchangeably installed and used and allow a calibration tool to be easily mounted to enable quick and accurate calibration, and includes a sleeve 1, a chuck device part 2, a holder part 3, and a calibration mounting part 4.

The sleeve 1 refers to a component that supports a shaft 71, 421 of a medical tool 7 such as a cutter 7a or a calibration tool 7b (to be described later), and is characterized in supporting various medical tools interchangeably unlike a conventional long and thin pipe that lacks compatibility and has weak bending rigidity.

In more detail, the sleeve 1 is roughly shaped like a round bar, and includes a hollow sleeve body 11 having a relatively large outer diameter in a portion for coupling with the chuck device part 2, a coupling male-thread portion 13 formed on an outer circumferential surface on one side, and a shaft insertion hole 12 formed penetrating in a longitudinal direction.

In addition, the sleeve 1 includes a sleeve cap 14 coupled to the shaft insertion hole 12 corresponding to an outer free end of the hollow sleeve body 11 by laser welding or the like joining method, a plurality of first support bearings 15 provided in the shaft insertion hole 12 being in contact with the sleeve cap 14 to support the shaft, a spacer 16 formed having a cylindrical shape and provided being in contact with the first support bearing 15, a plurality of second support bearings 17 provided being in contact with the spacer 16 and supporting the shaft, and an airtight member 18 inserted through a coupling female-thread portion 19 so as to be placed inside the shaft insertion hole 12.

Further, the shaft insertion hole 12 is formed with a fitting groove 121 to which the sleeve cap 14, the first and second support bearings 15 and 17, and the spacer 16 are fitted, a locking protrusion 122 to which the second support bearing 17 is locked, and an airtight member insertion groove 123 recessed at an inner end of the coupling female-thread portion 19 to insert the airtight member 18 therein.

The first and second support bearings 15 and 17 refer to components inserted in the front and rear of the fitting groove 121 with the spacer 16 therebetween to support the outer and inner portions of the shaft 71, and are provided as ball bearings. In this case, two ball bearings are arranged in succession to ensure sufficient durability even when the shaft rotates at high speed. In addition, as is publicly known, the ball bearing includes an inner ring centrally disposed inward, an outer ring concentrically disposed outside the inner ring, multiple balls interposed between the inner ring and the outer ring, and a bearing cover coupled to seal the balls. Further, the ball bearing is formed so that the inner diameter of the inner ring fits the outer diameter of a mounting large diameter portion of the cutter (to be described later).

The chuck device part 2 refers to a component to which the sleeve 1 including the shaft is fastened, and performs a clamping operation or an unclamping operation by pressing the shaft 71 of the medical tool as chucks 22 provided in the front of a chuck body 21 are brought together or separated.

In addition, the chuck device part 2 may employ a medical chuck adjustment device used in orthopedics, dentistry, etc. among well-known chuck devices, and thus detailed descriptions thereof will be omitted.

For example, the chuck device part 2 includes the chuck body 21 to which the sleeve 1 is coupled, the chucks 22 provided inside the chuck body 21 and selectively clamping the shaft 71 of the cutter 7a to clamp the surgical tool, and an operation force applying part (not shown) applying operation forces for the clamping and unclamping operations of the chucks 22.

In addition, the chuck body 21 is internally provided with a chuck moving member 24 from which the chuck 22 appears and disappears by the force of an internal spring as the operation force applying part (not shown) rotates forward and backward inside a round bar-shaped body approximately having a hollow. In more detail, when the operation force applying part (not shown) rotates forward, the chucks 22 move rearward and is brought together to press and clamp the shaft. When the operation force applying part rotates backward, the chucks move frontward and separate to unclamp the shaft.

Meanwhile, the holder part 3 refers to a component mounted to a distal end of a robot arm (not shown) and used to install the chuck device part 2 thereon, and includes a holder rod 31 roughly shaped like a rod, a holder head 32 formed below at a first end of the holder rod 31, a connection plate 33 formed above at a second end of the holder rod 31, and an arm connection member 34 connected to the connection plate 33 for the connection with the distal end of the robot arm.

The holder head 32 is formed as a ring body having a coupling hole in which a motor coupling member 37 for the connection of a motor 9 is inserted, and includes a sleeve supporter 35 protruding frontward to support the sleeve 1, and an operation handle 36 placed inside the sleeve supporter 35. The operation handle 36 serves to rotate the operation force applying part (not shown) that applies the operation force for the clamping and unclamping operations of the chucks 22 as rotating forward and backward.

Meanwhile, the calibration mounting part 4 is detachably provided in the sleeve 1 so that the calibration tool 7b such as a marker for calibrating the end of the cutter (drill) in artificial joint surgery using a robot can be installed.

In more detail, the calibration mounting part 4 includes a holding cap member 41 fastened to the sleeve 1, and a calibration mounting member 42 mounted to the sleeve 1 by the holding cap member 41.

The calibration mounting member 42 includes a calibration shaft 421 in which the marker is installed, and a position-setting member 422 formed in the calibration shaft 421 and seated on the holding cap member 41.

The calibration shaft 421 is formed in a bar-shaped structure such as a round bar having a predetermined length. According to this embodiment, the calibration may be easily performed using one calibration shaft 421 because a cutter having a head diameter of 5.0 mm for Uni-knee Arthroplasty (UKA) and a cutter having a head diameter of 3.2 mm for Guide Hole Resection (GHR) have the same overall length of 115.85 mm. A cutter having a head diameter of 6.2 mm for Total Knee Arthroplasty (TKA) has a longer length of 135.65 mm, and therefore the calibration may be performed using the same calibration shaft by inputting a value compensated for length difference to a robot system in advance to correct calibration data of the cutter of 6.2 mm.

The position-setting member 422 is formed on the outer circumferential surface of the calibration shaft 421, and shaped like a disc to be inserted and seated inside the holding cap member 41.

The holding cap member 41 includes a cap body 411 shaped like a cap with an anti-slip groove 416 recessed thereon and connected to a hollow sleeve body 44, a rod insertion hole 412 formed penetrating the cap body to insert the calibration shaft 421 therein, and a cap female thread 413 formed on the inner circumferential surface of the cap body to be fastened to the coupling male-thread portion 13 of the sleeve 1.

In addition, the holding cap member 41 may be formed with an inspection opening 415 to inspect the introduction of foreign substances by checking an alignment state between the sleeve 1 and the calibration mounting member 42 in the cap body 411.

For example, the inspection opening 415 is formed by cutting about 1/4 out of the cap body 411, and shows the interior to check the alignment state of the calibration mounting member 42. In other words, a gap formed when foreign substances are introduced between the inner surface of the position-setting member 422 and the end of the sleeve 1 is checkable with the naked eye through the inspection opening 415. When the gap is confirmed with the naked eye, the holding cap member 41 is separated from the sleeve, cleaned, and then reassembled, thereby performing the calibration accurately.

Further, the calibration mounting member 42 includes a cap separation preventing means 43 to prevent the holding cap member 41 from being separated from the calibration shaft 421.

The cap separation preventing means 43 may be variously configured without any particular limitations as long as it can prevent the separation of the holding cap member 41. According to this embodiment, the cap separation preventing means 43 includes a separation-preventing groove 431 recessed on the calibration shaft 421, and a separation-preventing piece 432 such as a C-ring to be press-fitted into the separation-preventing groove.

Meanwhile, the calibration mounting part 4 includes a mounting member rotation blocking portion 44 configured to block the movement of the calibration mounting member 42 for accurate and stable calibration.

The mounting member rotation blocking portion 44 includes a pin insertion groove 441 formed in the sleeve 1, and a rotation blocking pin 442 formed in the position-setting member and inserted in the pin insertion groove 441.

Here, the rotation blocking pin 442 includes two bar-shaped pins installed protruding from the position-setting member 422 at an angle of 180°.

In addition, the pin insertion groove 441 is formed in the sleeve 1 at a position corresponding to the rotation blocking pin 442, and structured to have a foreign substance discharging portion on one side to facilitate the discharge of the foreign substances. Here, the foreign substance discharging portion refers to a portion exposed to the outside by cutting an outer portion out of the pin insertion groove 441.

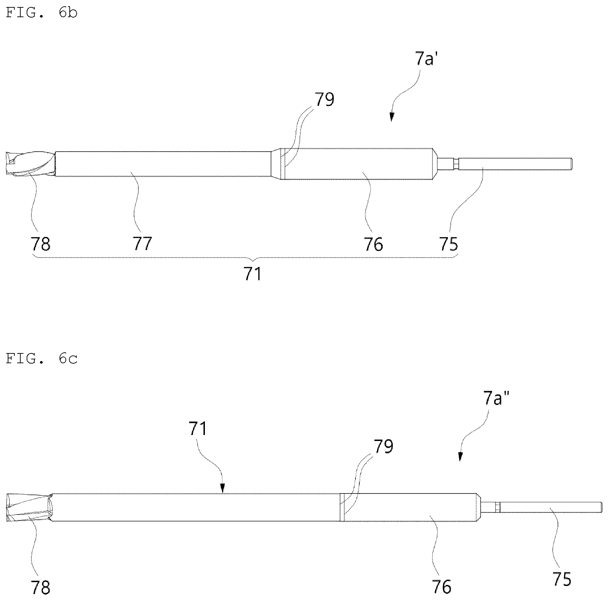

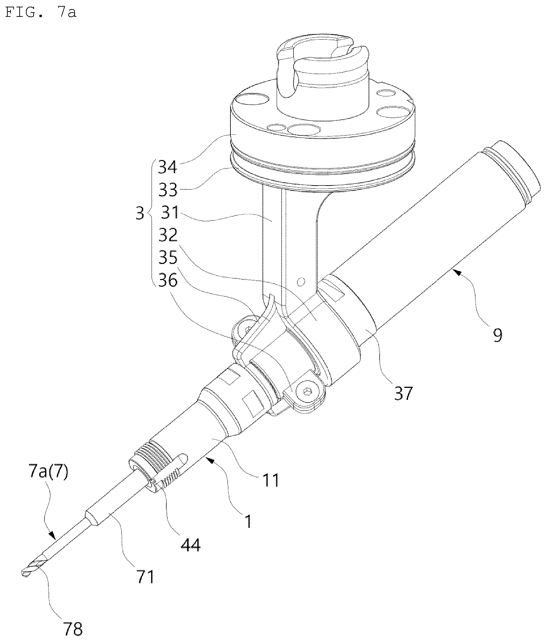

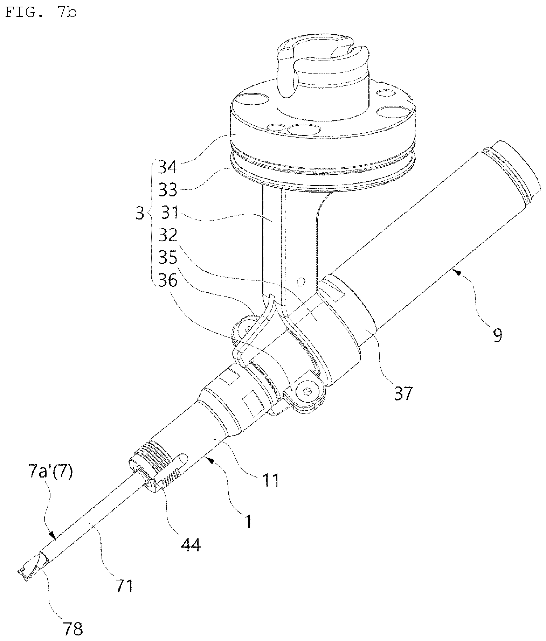

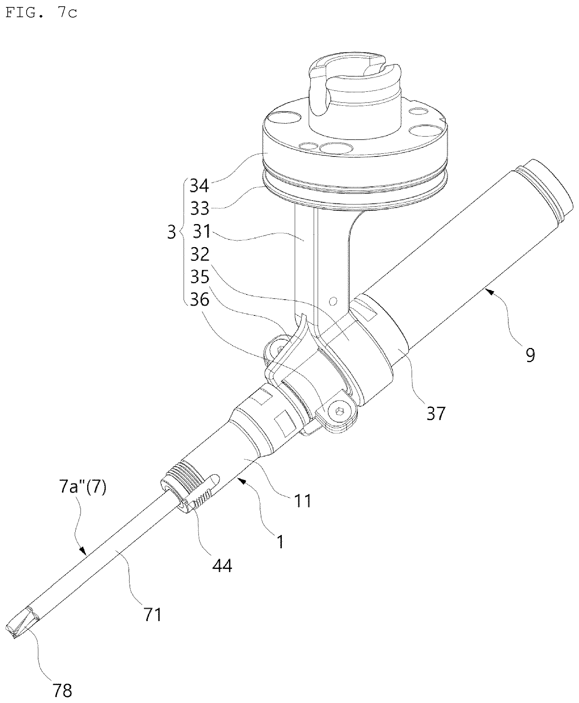

FIGS. 6A to 6C are front views showing cutters used as surgical tools in a calibration mounting part-equipped surgical device for a surgical robot according to an embodiment of the disclosure, in which FIG. 6A shows a cutter having a head diameter of 3.2 mm, FIG. 6B shows a cutter having a head diameter of 5.0 mm, and FIG. 6C shows a cutter having a head diameter of 6.2 mm. FIGS. 7A to 7C are perspective views of a calibration mounting part-equipped surgical device for a surgical robot installed with the cutters as the surgical tools according to an embodiment of the disclosure, in which FIG. 7A shows the installed state of a cutter having a head diameter of 3.2 mm, FIG. 7B shows the installed state of a cutter having a head diameter of 5.0 mm, and FIG. 7C shows the installed state of a cutter having a head diameter of 6.2 mm.

Referring to FIGS. 6A to 7C, a calibration mounting part-equipped surgical device for a surgical robot according to an embodiment of the disclosure includes the sleeve 1 with the hollow sleeve body 11 having a relatively large outer diameter rather than a long and thin pipe, so that various types of cutters can be applied thereto.

For example, as shown in FIGS. 6A to 6C, the cutter 7a may employ a cutter 7a having a head diameter of 3.2 mm, a cutter 7a' having a head diameter of 5.0 mm, and a cutter 7a" having a head diameter of 6.2 mm.

These cutters 7a are different in the outer diameter and shape of a cutter head 78, and have the same or different overall length of the shaft 71, but are commonly provided with a round bar-shaped small diameter portion 75 having a relatively small diameter so that the shaft can be inserted in the chuck device part 2.

Each of the cutter 7a having a head diameter of 3.2 mm, the cutter 7a' having a head diameter of 5.0 mm, and the cutter 7a" having a head diameter of 6.2 mm includes a mounting large diameter portion 76 formed in contact with the small diameter portion, inserted in the sleeve 1, and having a relatively large outer diameter compared to that of the small diameter portion; and a middle diameter portion 77 extending in contact with the mounting large diameter portion, formed with a cutter head 78 at the end thereof, and having an outer diameter larger than that of the small diameter portion and smaller than that of the mounting large diameter portion.

In addition, each of the foregoing cutters is marked with two mounting check bands 79 on the mounting large diameter portion 76 so that the mounting can be checked without using a separate cutter gauge. In this case, the cutter 7a, 7a', or 7a" is considered to be normally mounted when the outer one of the two mounting check bands 79 is exposed and checked. Therefore, when the two mounting check bands 79 are both hidden or exposed, the cutter 7a, 7a' or 7a" is considered to be abnormally mounted and thus its mounting state is adjusted to expose one mounting check band before use.

As described above, the foregoing cutters include the mounting large diameter portion 76 to be mounted to the shaft insertion hole 12 of the sleeve 1, and are thus easily mounted to and interchangeably used in one surgical device as shown in FIGS. 7A to 7C.

Meanwhile, the calibration tool 7b refers to an optical marker detachably provided in the sleeve 1 and applied to an Optical Tracking System (OTS), and includes a marker body 75 formed with a shaft holding hole (not shown) in which the calibration shaft 421 is inserted, and a tightening means 76 such as a fastening bolt to secure the calibration shaft 421 inserted in the shaft holding hole. In addition, the marker body 75 includes a plurality of position transmitting units 77 roughly shaped like balls to reflect or transmit a position signal to the OTS. For reference, the OTS refers to a system that tracks the marker with a plurality of infrared cameras, and converts the distance through the triangulation, thereby tracking the position and posture in a three-dimensional space in real-time. The tracking principle of the OTS has been widespread, and thus detailed descriptions thereof will be omitted for simplicity of description.

Below, operations of a calibration mounting part-equipped surgical device for a surgical robot according to an embodiment of the disclosure will be described in brief.

After the arm connection member 34 of the foregoing calibration mounting part-equipped surgical device for the surgical robot is connected to a clamping member (not shown) provided in the robot arm (not shown) of the orthopedic surgical robot, the cutter 7a or the like medical tool 7 is inserted and installed into the sleeve 1, and the robot arm of the surgical robot operating based on information input to a computer is positioned at a surgical site, surgery is performed in given order.

Specifically, when a motor 9 operates based on the information input to the computer to rotate the cutter 7a mounted to the chuck device part 2, the cutter head 78 formed at the end of the cutter 7a cuts a surgical site of a kneecap and an artificial knee joint (implant) is mounted in given order, thereby performing a surgical procedure.

The foregoing cutter 7a is stably rotated during a surgical process because the shaft 71 is supported by the sleeve 1. The sleeve 1 according to the disclosure includes the hollow sleeve body 11 having a relatively large outer diameter rather than a conventional thin and long pipe, so that the cutter having a head diameter of 3.2 mm, the cutter having a head diameter of 5.0 mm, and the cutter having a head diameter of 6.2 mm can be easily replaced and used as shown in FIGS. 7A to 7C without replacing the sleeve every time when the type of cutter is changed.

Meanwhile, a calibration mounting part-equipped surgical device for a surgical robot according to the disclosure may use the calibration mounting part 4 to easily install the calibration tool 7b such as the marker for calibrating the end of the cutter during the artificial joint surgery using the robot.

In more detail, the calibration mounting part 4 is assembled in such a manner that the cutter 7a is first separated from the chuck device part 2, the calibration mounting member 42 is inserted in the holding cap member 41, and the holding cap member 41 is fastened to the coupling male-thread portion 13 of the sleeve 1. In this assembling process, when the rotation blocking pin 442 formed in the position-setting member 422 is inserted in the pin insertion groove 441 formed in the sleeve 1, the movement such as the rotation of the calibration mounting member 42 in which the calibration tool 7b is installed is fundamentally blocked, thereby having an advantage of enabling accurate calibration.

In addition, the foreign substance discharging portion, i.e., the exposed portion is formed by cutting an outer portion out of the pin insertion groove 441 formed in the sleeve 1, thereby having an advantage of easily discharging foreign substances to the outside even though the foreign substances are introduced due to the operation of the cutter during the surgery.

Further, the inspection opening 415 is formed in the holding cap member 41, and thus, when a gap formed by foreign substances introduced between the inner surface of the position-setting member 422 and the end of the sleeve 1 is confirmed with the naked eye, the holding cap member 41 is separated from the sleeve, cleaned, and then reassembled, thereby performing the calibration accurately.

The terms "comprise," "configure" and/or "have" specify the presence of stated components, unless there is a clearly different meaning in the disclosure, but do not preclude the presence thereof and should be construed to further include other components. Unless otherwise defined, all terms including technical or scientific terms used herein have the same meaning as commonly understood by those skilled in the art to which the disclosure pertains. General terms that are defined in a dictionary shall be construed to have meanings that are consistent in the context of the relevant art, and will not be interpreted as having an idealistic or excessively formalistic meaning unless clearly defined in the disclosure.

Although the configurations and operations of a calibration mounting part-equipped surgical device for a surgical robot according to an embodiment of the disclosure have been described above, it will be understood by a person having ordinary knowledge in the art that the embodiments are merely illustrative and may be partially substituted and modified without departing from the scope of the disclosure.

### [INDUSTRIAL APPLICABILITY]

According to the disclosure, a calibration mounting part-equipped surgical device for a surgical robot, which is applicable to artificial joint surgery using the robot, is appliable to various surgical robots in a medical field as well as an artificial joint surgical robot, so that a medical tool can be interchangeably installed and used, and a calibration tool can be easily installed to perform accurate calibration.

## Claims

1. A surgical device for a surgical robot, comprising:
a sleeve supporting a medical tool comprising a shaft;
a chuck device part coupling with the sleeve comprising the shaft;
a holder part installed with the chuck device part and mounted to a distal end of a robot arm; and
a calibration mounting part detachably provided in the sleeve and installed with a calibration tool.

2. The surgical device of claim 1, wherein the calibration mounting part comprises:
a holding cap member fastened to the sleeve; and
a calibration mounting member mounted to the sleeve by the holding cap member.

3. The surgical device of claim 2, wherein
the calibration mounting member comprises a bar-shaped calibration shaft in which a marker is installed, and a position-setting member formed in the calibration shaft and seated on the holding cap member,
the sleeve comprises a hollow sleeve body formed with a coupling male-thread portion formed on an outer circumferential surface on one side, and a shaft insertion hole formed penetrating in a longitudinal direction, and
the holding cap member comprises a cap body shaped like a cap and connected to the hollow sleeve body, a rod insertion hole formed in the cap body to insert the calibration shaft therein, and a cap female thread formed on an inner circumferential surface of the cap body to be fastened to the coupling male-thread portion.

4. The surgical device of claim 3, further comprising a mounting member rotation blocking portion configured to block the movement of the calibration mounting member.

5. The surgical device of claim 4, wherein the mounting member rotation blocking portion comprises:
a pin insertion groove formed in the sleeve; and
a rotation blocking pin formed in the position-setting member and inserted in the pin insertion groove.

6. The surgical device of claim 5, wherein the pin insertion groove is structured to comprise a foreign substance discharging portion on one side to facilitate the discharge of foreign substances.

7. The surgical device of claim 5, wherein
the calibration mounting member comprises a cap separation preventing means configured to prevent the holding cap member from being separated from the calibration shaft,
the position-setting member is shaped like a disc to be inserted in the holding cap member,
the rotation-blocking pin comprises a plurality of rotation-blocking pins protruding from the disc, and
the holding cap member is formed with an inspection opening to inspect an alignment state between the sleeve and the calibration mounting member in the cap body.

8. The surgical device of claim 3, wherein
the sleeve comprises a sleeve cap coupled to the shaft insertion hole corresponding to an outer free end of the hollow sleeve body; a plurality of first support bearings provided in the shaft insertion hole being in contact with the sleeve cap and supporting the shaft; a spacer formed having a cylindrical shape and provided being in contact with the first support bearing; a plurality of second support bearings provided being in contact with the spacer and supporting the shaft; and an airtight member inserted through a coupling female-thread portion formed inside the shaft insertion hole, and
the shaft insertion hole comprises a fitting groove to which the sleeve cap, the first and second support bearings, and the spacer are fitted; a locking protrusion to which the second support bearing is locked; and an airtight member insertion groove recessed at an inner end of the coupling female-thread portion and inserting the airtight member therein.
